# EUROPÄISCHE PATENTSCHRIFT

(11) **EP 0 941 216 B1**
(45) Veröffentlichungstag und Bekanntmachung des Hinweises auf die Patenterteilung: **24.10.2001**
(21) Anmeldenummer: 97953699.2
(22) Anmeldetag: 25.11.1997
(51) Int. Cl.: C07C 261/02, C08G 8/28

(54) **VERFAHREN ZUR HERSTELLUNG VON ARYLCYANATEN**
METHOD FOR PRODUCING ARYL CYANATES
PROCEDE POUR LA PREPARATION DE CYANATES D'ARYLE

(30) Priorität: 29.11.1996 CH 293696
(43) Veröffentlichungstag der Anmeldung: 15.09.1999
(73) Patentinhaber: LONZA AG, CH-3945 Gampel/Wallis (CH)
(72) Erfinder: FALCHETTO, Alessandro, I-28030 Montecrestese (IT)
(86) Internationale Anmeldenummer: EP9706579
(87) Internationale Veröffentlichungsnummer: WO9823584

(56) Entgegenhaltungen:
- DE-A- 2 533 322
- DE-A- 19 519 102

## Beschreibung

Die vorliegende Erfindung betrifft ein Verfahren zur Herstellung von Arylcyanaten durch Umsetzung von mehrwertigen Phenolen mit Chlor- oder Bromcyan in Gegenwart von tertiären Aminen.

Die Cyansäureester mehrwertiger Phenole, hier und im folgenden kurz als Arylcyanate bezeichnet, sind Ausgangsmaterialien für die Herstellung hochtemperaturbeständiger Duroplaste mit Triazinstruktur (s. z. B. DE-A 17 20 663). Unter mehrwertigen Phenolen sind hier und im folgenden insbesondere solche Verbindungen zu verstehen, in denen zwei oder mehrere mit jeweils einer Hydroxygruppe substituierte Benzolringe über direkte Bindungen, niedere zwei- oder mehrwertige acyclische Kohlenwasserstoffreste, zwei- oder mehrwertige mono- oder polycyclische aromatische oder cycloaliphatische Reste, Heteroatome oder aus den vorgenannten Resten zusammengesetzte komplexe Gruppen miteinander verbunden sind. Hierzu zählen beispielsweise die verschiedenen als Bis- und Trisphenole bekannten Verbindungen wie Bisphenol A und die unter der Bezeichnung Novolake bekannten oligomeren Kondensationsprodukte aus Formaldehyd und Phenolen oder Kresolen.

Die genannten Arylcyanate werden üblicherweise dadurch hergestellt, dass das entsprechende mehrwertige Phenol oder sein Addukt bzw. Salz mit einem tertiären Amin, beispielsweise Triethylamin, in einem nichtwässrigen Lösungsmittel wie beispielsweise Aceton mit einem Halogencyan, insbesondere Chlor- oder Bromcyan, bei möglichst niedriger Temperatur umgesetzt wird (s. z. B. DE-A 195 19 102). Zur Bindung des entstehenden Halogenwasserstoffs dient dabei das im Phenol-Amin-Addukt vorhandene oder - bei Einsatz von freiem Phenol - als solches zugesetzte tertiäre Amin. Hierdurch entsteht in molaren Mengen ein tertiäres Ammoniumhalogenid, das vollständig entfernt werden muss, insbesondere dann, wenn das hergestellte Arylcyanat nicht durch Kristallisation oder Destillation gereinigt werden kann. Diese Entfernung geschieht üblicherweise durch Extraktion mit Wasser, gegebenenfalls nach vorheriger Filtration oder Zentrifugation von ausgefallenem Ammoniumhalogenid. Hierfür sind aber nur solche Lösungsmittel gut geeignet, die mit Wasser nicht mischbar sind. Bei Verwendung wassermischbarer Lösungsmittel wie beispielsweise Aceton fallen nämlich wässrige Lösungsmittelgemische an, die nur mit grossem Aufwand wieder aufgearbeitet werden können und deshalb meistens als Abfall entsorgt werden müssen. Aber auch bei Verwendung von nicht mit Wasser mischbaren Lösungsmitteln ergeben sich häufig Probleme bei der Phasentrennung, durch im Extraktionswasser verbleibende Lösungsmittelspuren und durch Wasserspuren in der organischen Phase, die eine zusätzliche Trocknung erforderlich machen können.

Aufgabe der vorliegenden Erfindung war daher, ein Verfahren mit einer alternativen Aufarbeitungsmethode zur Verfügung zu stellen, das auch bei der Verwendung wassermischbarer Lösungsmittel für die Durchführung der Reaktion eine einfache und vollständige Entfernung des anfallenden Ammoniumhalogenids erlaubt, ohne problematische Abfälle zu erzeugen.

Erfindungsgemäss wird diese Aufgabe durch das Verfahren nach Patentanspruch 1 gelöst.

Es wurde gefunden, dass durch eine einfache Behandlung des auf an sich bekannte Weise erhaltenen Reaktionsgemisches mit Kationen- und Anionenaustauschem das Ammoniumhalogenid praktisch vollständig entfernt werden kann. Hierbei kann entweder die gesamte Menge an Ammoniumhalogenid oder der nach teilweiser Entfernung durch eine andere Methode verbleibende Rest durch die Ionenaustauscherbehandlung entfernt werden. Vorzugsweise wird die Hauptmenge des Ammoniumhalogenids bereits vor der Ionenaustauscherbehandlung abgetrennt, indem ein Lösungsmittel eingesetzt wird, in dem das Ammoniumhalogenid schwer löslich ist und das Reaktionsgemisch nach Beendigung der Reaktion filtriert oder zentrifugiert wird.

Das erfindungsgemässe Verfahren wird vorzugsweise derart durchgeführt, dass das gegebenenfalls filtrierte oder zentrifugierte Reaktionsgemisch zunächst mit einem stark sauren Kationenaustauscher und anschliessend mit einem schwach basischen Anionenaustauscher behandelt wird. Es liegt jedoch ebenfalls im Rahmen der Erfindung, eine andere Vorgehensweise zu wählen, beispielsweise ein Mischbett aus Kationen- und Anionenaustauscher einzusetzen. Als Kationenaustauscher eignen sich beispielsweise stark saure Ionenaustauscher wie Lewatit® S 100 (Hersteller: Bayer AG), als Anionenaustauscher beispielsweise schwach basische Ionenaustauscher wie Lewatit® MP 62. (Bei Verwendung stark basischer Ionenaustauscher besteht bei längerer Einwirkungszeit die Gefahr der Hydrolyse der Cyanatgruppen.) Wesentlich ist, dass sich die polymeren Basismaterialien der Ionenaustauscher in den im erfindungsgemässen Verfahren verwendeten Lösungsmitteln nicht auflösen oder übermässig quellen. Die Ionenaustauscherbehandlung wird vorteilhaft derart durchgeführt, dass das gegebenenfalls filtrierte oder zentrifugierte Reaktionsgemisch jeweils durch eine Säulenpackung des Ionenaustauschers perkoliert wird; es ist aber auch möglich, den bzw. die Ionenaustauscher im Reaktionsgemisch bzw. Filtrat/Zentrifugat zu suspendieren und nach erfolgtem Austausch durch Filtration oder Zentrifugation abzutrennen.

Das erfindungsgemässe Verfahren wird vorzugsweise in einem Lösungsmittel aus der Gruppe bestehend aus Dichlormethan, Di-C₁₋₄-alkylketonen, C₁₋₄-Alkyl-C₂₋₄-alkanoaten, Tetrahydrofuran und Acetonitril oder einem Gemisch dieser Lösungsmittel durchgeführt. Unter Di-C₁₋₄-alkylketonen sind hier Ketone mit zwei gleichen oder verschiedenen linearen oder verzweigten C₁₋₄-Alkylgruppen wie beispielsweise Aceton (Dimethylketon), Butanon (Methylethylketon) oder Diisopropylketon zu verstehen. Unter C₁₋₄-Alkyl-C₂₋₄-alkanoaten sind Ester der Essig-, Propion- oder (Iso-) Buttersäure mit C₁₋₄-Alkoholen zu verstehen.

Besonders bevorzugte Lösungsmittel sind Dichlormethan, Aceton und Butylacetat.

Die weitere Aufarbeitung nach der Ionenaustauscherbehandlung kann auf an sich bekannte Weise erfolgen. Vorteilhaft wird zunächst das Lösungsmittel weitgehend abdestilliert. Dieses kann anschliessend beispielsweise durch Rektifikation aufgearbeitet und in das Verfahren zurückgeführt werden. Kristallisierbare Arylcyanate wie beispielsweise das Bisphenol A-Dicyanat können direkt aus der im Sumpf der Lösungsmitteldestillation anfallenden konzentrierten Lösung kristallisiert oder als Schmelze isoliert werden. Nicht kristallisierbare Arylcyanate wie beispielsweise Novolakcyanate werden zweckmässig zur Abtrennung von flüchtigen Verunreinigungen wie Dialkylcyanamiden und Lösungsmittelresten einer Vakuumdestillation, beispielsweise in einem Fallfilmverdampfer, unterzogen.

Die mit den tertiären Ammoniumionen und den Halogenidionen beladenen Ionenaustauscher können nach Erschöpfung ihrer Austauschkapazität wieder auf übliche Weise mit einer starken Säure (Kationenaustauscher) bzw. einer mittelstarken bis starken Base (Anionenaustauscher) regeneriert und mehrfach verwendet werden.

Die Umsetzung der mehrwertigen Phenole mit dem Halogencyan kann sowohl diskontinuierlich, beispielsweise in einem Rührkessel, als auch kontinuierlich erfolgen. Ein kontinuierliches Verfahren ist beispielsweise in DE-A 195 19 102 beschrieben.

Die erfindungsgemäss herstellbaren Arylcyanate können beispielsweise durch die allgemeine Formel 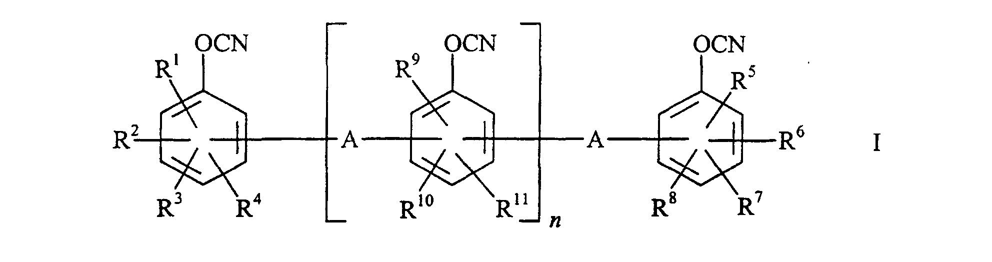 beschrieben werden.

Hierin ist *n* eine ganze Zahl von 0 bis 20, vorzugsweise 0 bis 10. Es fallen damit sowohl bifunktionelle als auch polyfunktionelle (oligomere) Arylcyanate unter Formel I. Letztere leiten sich beispielsweise von mehrwertigen Phenolen des Novolak-Typs ab.

A kann sowohl eine direkte chemische Bindung als auch eine zweiwertige Gruppe sein. Mögliche zweiwertige Gruppen sind beispielsweise -O-, -S(O)ₓ- mit *x* = 0 bis 2, -C(O)-, -OC(O)O-, lineare oder verzweigte gegebenenfalls fluorsubstituierte C₁₋₁₀-Alkandiylgruppen, zweiwertige mono- oder polycyclische aromatische Reste, die gegebenenfalls mit einer oder mehreren C₁₋₄-Alkylgruppen und/oder einem oder mehreren Halogenatomen substituiert sein können, zweiwertige mono- oder polycyclische cycloaliphatische Reste, die gegebenenfalls mit einer oder mehreren C₁₋₄-Alkylgruppen substituiert sein können, sowie die aus zweien oder mehreren der vorstehend genannten zweiwertigen Gruppen zusammengesetzten zweiwertigen Gruppen. Falls *n* grösser als Null ist und somit mehrere zweiwertige Gruppen A vorhanden sind, können diese gleich oder verschieden sein. Vorzugsweise sind die Gruppen A gleich.

Die Substituenten R¹ bis R¹¹ können gleich oder verschieden sein und neben Wasserstoff jeweils Halogen, C₁₋₄-Alkoxy, C₁₋₄-Alkylthio, C₁₋₄-Alkoxycarbonyl oder C₁₋₄-Alkyl, welches gegebenenfalls vollständig oder teilweise fluorsubstituiert sein kann, sein.

Sowohl die zweiwertigen Gruppen A als auch die Substituenten R¹ bis R¹¹ können jeweils in ortho-, meta- oder para-Stellung zu den Cyanatgruppen stehen, wobei das Substitutionsmuster an den einzelnen aromatischen Ringen sowohl innerhalb eines Moleküls als auch von Molekül zu Molekül verschieden sein kann.

Unter dem Begriff "Alkandiylgruppe" sind hier und im folgenden sowohl solche Gruppen, deren freie Valenzen vom selben Kohlenstoffatom ausgehen, wie beispielsweise Methylen, Ethylen oder 1-Methylethyliden (= "Isopropyliden"), als auch solche deren freie Valenzen von verschiedenen Kohlenstoffatomen ausgehen, wie beispielsweise 1,2-Ethandiyl (= "Ethylen") oder 1,3-Propandiyl (= "Trimethylen"), zu verstehen.

Ein Beispiel für eine fluorsubstituierte Alkandiylgruppe ist 1-(Trifluormethyl)-2,2,2-trifluorethyliden.

Unter dem Begriff "polycyclisch" sind hier und im folgenden alle cyclischen Strukturen, die mindestens zwei Ringe aufweisen, zu verstehen. Hierunter fallen einfach verbundene Ringe wie zum Beispiel Biphenyl, Spiro-Verbindungen, kondensierte Ringsysteme wie beispielsweise Naphthalin, verbrückte Ringsysteme wie beispielsweise Norbornan und Systeme, die mehrere dieser Merkmale aufweisen wie beispielsweise Octahydro-4,7-methanoinden.

Zweiwertige aromatische Reste sind beispielsweise *o*-, *m-* und *p*-Phenylen und die verschiedenen isomeren Naphthylene und Biphenylylene.

Bei zweiwertigen cycloaliphatischen Resten können die beiden freien Valenzen vom selben Kohlenstoffatom ausgehen, wie beispielsweise bei Cyclohexyliden, oder von verschiedenen Kohlenstoffatomen wie beispielsweise bei Octahydro-4,7-methanoindendiyl. Zusammengesetzte zweiwertige Gruppen sind beispielsweise solche der Formeln

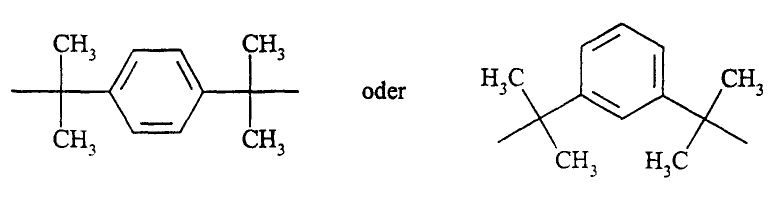

Insbesondere eignet sich das erfindungsgemässe Verfahren zur Herstellung der folgenden Arylcyanate:

### 4,4'-Thiodiphenylcyanat der Formel

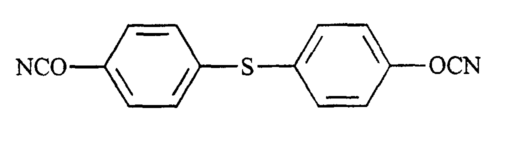

### 4,4'-Methylenbis(2,6-dimethylphenylcyanat) der Formel

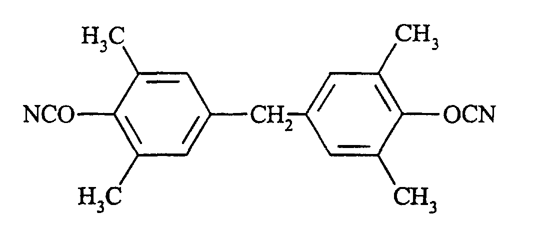

### Arylcyanate der Formel

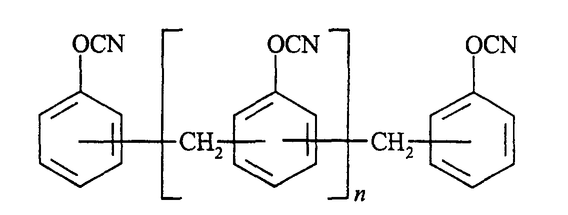

### 4,4'-Ethylidendiphenylcyanat der Formel

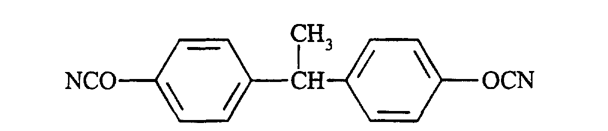

### 2,2-Bis(4-cyanatophenyl)propan der Formel

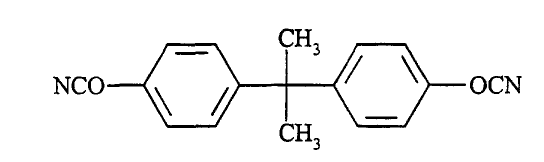

### 4,4'-[Bis(trifluormethyl)methylen]diphenylcyanat der Formel

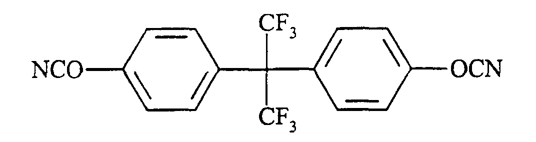

### Arylcyanate der Formel

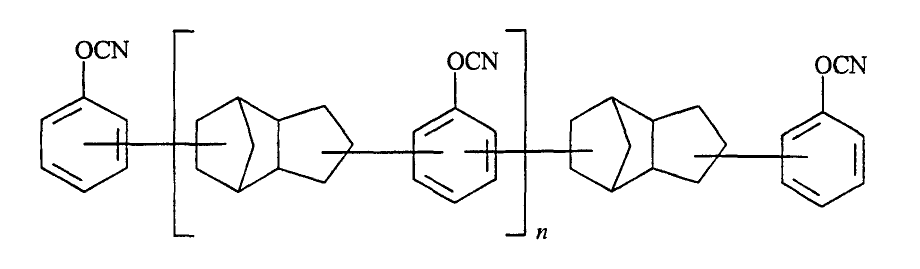

### 4,4'-(1,3-Phenylendiisopropyliden)diphenylcyanat der Formel

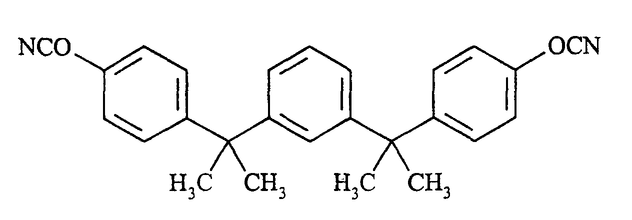

### 4,4'-(1,4-Phenylendiisopropyliden)diphenylcyanat der Formel

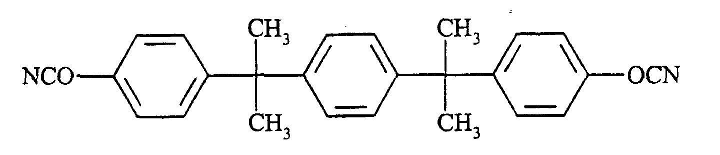

Die nachfolgenden Beispiele verdeutlichen die Durchführung des erfindungsgemässen Verfahrens, ohne dass darin eine Einschränkung zu sehen ist.

### Beispiel 1

### Herstellung eines Novolak-Cyanates

In 564 g Butylacetat wurden 100 g Novolak (Durite® SD-333A, Hersteller: Borden Chem. Co.) gelöst. Die Lösung wurde auf ca. -15 °C gekühlt, dann wurden 67 g gasförmiges Chlorcyan eingeleitet. Anschliessend wurden unter Rühren innerhalb von ca. 30 min 104 g Triethylamin zugetropft, wobei die Temperatur unter-10 °C gehalten wurde. Nach weiteren 30 min bei dieser Temperatur wurde die Kühlung entfernt und das Reaktionsgemisch filtriert. Eine durch Eindampfen des Filtrats erhaltene Probe enthielt 190 ppm Triethylamin und 74 ppm Chlorid. Das Filtrat wurde nacheinander über eine Säule aus 100 ml Lewatit® S100 (Kationenaustauscher, H⁺-Form) und eine Säule aus 100 ml Lewatit® MP62 (Anionenaustauscher, OH⁻-Form) gegeben. Die Ionenaustauscher waren zuvor mit 5%iger Salzsäure bzw. 4%iger Natronlauge regeneriert, mit Wasser neutral gespült, mit Aceton entwässert und schliesslich mit Butylacetat gespült worden. Anschliessend wurde zunächst das Lösungsmittel bei 70 °C Badtemperatur im Wasserstrahlvakuum abdestilliert. Das so erhaltene Material hatte eine Gelzeit von 30 bis 47 min bei 200 °C, einen Triethylamingehalt von 31 ppm und einen Chloridgehalt von 4 ppm. Schliesslich wurden bei 1 mbar und 130 °C im Fallfilmverdampfer flüchtige Verunreinigungen (u. a. Lösungsmittelreste, freies Triethylamin, Diethylcyanamid) entfernt.

Das so erhaltene Produkt hatte eine Gelzeit von 50 min bei 200 °C und eine Viskosität von 284 mPa·s.

### Beispiel 2

### Herstellung von 2,2-Bis(4-cyanatophenyl)propan (Bisphenol A-Dicyanat)

In 480 g Aceton wurden 150 g Bisphenol A gelöst. Die Lösung wurde auf-25 bis -20 °C abgekühlt, dann wurden bei dieser Temperatur innerhalb von 15 min 88 g Chlorcyan eingeleitet. Anschliessend wurden innerhalb von ca. 1 h 136 g Triethylamin zugetropft, wobei die Temperatur bei ca. -15 °C gehalten wurde und Triethylammoniumchlorid ausfiel. Nach weiteren 30 min wurde die Kühlung abgestellt, das Reaktionsgemisch filtriert und der Filterkuchen mit Aceton gewaschen. Die vereinigten Filtrate wurden in zwei Teile aufgeteilt (je ca. 600 ml). Ein Teil wurde erfindungsgemäss zuerst mit 50 g Lewatit® S100 (Kationenaustauscher, H⁺-Form) und dann (nach Abfiltrieren des Ionenaustauschers) mit 50 g Amberlite® IRA-68 (stark basischer Anionenaustauscher, OH⁻-Form) gerührt und schliesslich nochmals filtriert. Die so erhaltene Lösung wurde im Vakuum auf ca. 200 ml eingeengt und dann mit 250 ml Wasser versetzt. Das restliche Aceton wurde abdestilliert, das ausgefallene Produkt abfiltriert und bei 50 °C 24 h im Vakuum getrocknet.

Der Chloridgehalt des so erhaltenen Produkts betrug 2 ppm.

### Beispiel 3

### Herstellung eines Novolak-Cyanates

In einem doppelwandigen Reaktionsgefäss wurden bei Raumtemperatur 103 g (1 Äquivalent) Novolak (Durite® SD-1711, Hersteller: Borden Chem. Co.; entspricht Formel I mit A = CH₂, R¹ bis R¹¹ = H, *n* = 1 bis 10) in 422 g Aceton gelöst. Die Lösung wurde auf ca. -15 °C gekühlt, dann wurden innerhalb von 15-20 min 66,4 g (1,06 mol) gasförmiges Chlorcyan eingeleitet, wobei die Temperatur auf-18 bis -14 °C gehalten wurde. Anschliessend wurden unter Rühren innerhalb von ca. 30 min 103,4 g (1,022 mol) Triethylamin zugetropft, wobei die Temperatur bei -19 bis -12 °C gehalten wurde. Dann wurde die Kühlung soweit reduziert, dass sich das Reaktionsgemisch innerhalb von ca. 30 min auf -5 °C erwärmte. Nach weiteren 30 min bei dieser Temperatur wurde das Gemisch wieder auf-15 °C abgekühlt. Die so erhaltene Suspension wurde bei dieser Temperatur durch eine Glasfritte filtriert. Der Filterkuchen wurde mit 3 × 50 ml Aceton von -15 °C gewaschen und getrocknet. (Das so erhaltene Triethylammoniumchlorid wurde zur Rückgewinnung des Triethylamins eingesetzt.) Das Filtrat wurde mit den Waschlösungen vereinigt und bei Raumtemperatur mit einer Durchflussrate von 200 ml/min nacheinander durch je 50 ml eines stark sauren Kationenaustauschers (Lewatit® S 100) und eines schwach basischen Anionenaustauschers (Lewatit® MP 62) filtriert. Anschliessend wurden beide Austauschersäulen mit 2 × 50 ml Aceton gewaschen. Durch die Ionenaustauscherbehandlung wurde der Chloridgehalt der Lösung von 297 ppm auf 58 ppm und der Triethylamingehalt von 572 ppm auf 6 ppm reduziert. Das Lösungsmittel wurde am Rotationsverdampfer abdestilliert, wobei die Badtemperatur von 45 °C auf 60 °C erhöht und der Druck von 500 mbar auf 50 mbar reduziert wurde. Der Rückstand wurde mit 11,4 g Cyclohexanon (als Schleppmittel) gründlich vermischt und dann zur Entfernung des Diethylcyanamids und des Cyclohexanons bei 130 °C Filmtemperatur und ca. 2 mbar durch einen Fallfilmverdampfer gegeben. Die Vorlage wurde dabei auf 80 °C erwärmt, um das Produkt flüssig zu halten.

Anschliessend wurde das viskose Produkt auf eine gekühlte Stahlplatte gegossen und die so erhaltene erstarrte Masse zerkleinert.
Ausbeute: 83,3 g
Gelzeit: 10-20 min bei 200 °C
Diethylcyanamid: ≤0,1%

### Vergleichsbeispiel

### Herstellung von 2,2-Bis(4-cyanatophenyl)propan

Die zweite Hälfte des Filtrats aus Beispiel 2 wurde direkt mit 250 ml Wasser versetzt und das Aceton vollständig abdestilliert. Das ausgefallene Produkt wurde abfiltriert und bei 50 °C 24 h im Vakuum getrocknet.

Der Chloridgehalt des so erhaltenen Produkts betrug 14 ppm.

## Patentansprüche

1. Verfahren zur Herstellung von Arylcyanaten durch Umsetzung mehrwertiger Phenole mit einem Halogencyan in Gegenwart eines tertiären Amins und eines nichtwässrigen Lösungsmittels bei tiefer Temperatur und anschliessende Entfernung des gebildeten tertiären Ammoniumhalogenids aus dem Reaktionsgemisch, **dadurch gekennzeichnet, dass** die Entfernung des Ammoniumhalogenids ganz oder teilweise durch Behandlung des nichtwässrigen Reaktionsgemisches mit Kationen- und Anionenaustauschern durchgeführt wird.

2. Verfahren nach Anspruch 1, **dadurch gekennzeichnet, dass** als Lösungsmittel ein Lösungsmittel eingesetzt wird, in welchem das gebildete Ammoniumhalogenid schwer löslich ist und die Hauptmenge des Ammoniumhalogenids vor der Ionenaustauscherbehandlung durch Filtration oder Zentrifugation abgetrennt wird.

3. Verfahren nach Anspruch 1 oder 2, **dadurch gekennzeichnet, dass** das gegebenenfalls filtrierte oder zentrifugierte Reaktionsgemisch zunächst mit einen stark sauren Kationenaustauscher und anschliessend mit einem schwach basischen Anionenaustauscher behandelt wird.

4. Verfahren nach einem der Ansprüche 1 bis 3, **dadurch gekennzeichnet, dass** als Lösungsmittel ein Lösungsmittel aus der Gruppe bestehend aus Dichlormethan, Di-C₁₋₄-alkylketonen, C₁₋₄-Alkyl-C₂₋₄-alkanoaten, Tetrahydrofuran und Acetonitril eingesetzt wird.

5. Verfahren nach Anspruch 4, **dadurch gekennzeichnet, dass** als Lösungsmittel Dichlormethan, Aceton oder Butylacetat eingesetzt wird.

6. Verfahren nach einem der Ansprüche 1 bis 5, **dadurch gekennzeichnet, dass** ein Arylcyanat der allgemeinen Formel 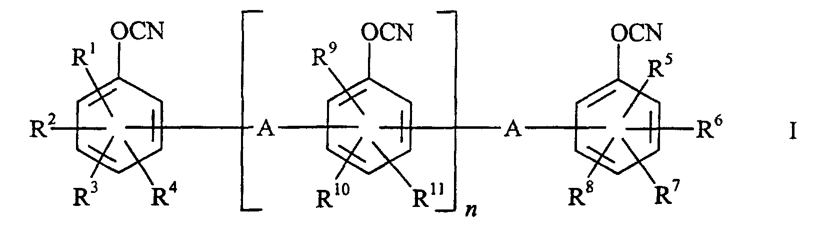 worin *n* eine ganze Zahl von 0 bis 20 ist, die Symbole A jeweils für eine direkte chemische Bindung oder eine zweiwertige Gruppe stehen und R¹ bis R¹¹ unabhängig voneinander Wasserstoff, Halogen, C₁₋₄-Alkoxy, C₁₋₄-Alkylthio, C₁₋₄-Alkoxycarbonyl oder gegebenenfalls vollständig oder teilweise fluorsubstituiertes C₁₋₄-Alkyl sind, hergestellt wird.

## Claims

1. Process for the preparation of aryl cyanates by reaction of polyhydric phenols with a cyanogen halide in the presence of a tertiary amine and a nonaqueous solvent at low temperature and subsequent removal of the formed tertiary ammonium halide from the reaction mixture, **characterized in that** some or all of the ammonium halide is removed by treatment of the nonaqueous reaction mixture with cation and anion exchangers.

2. Process according to Claim 1, **characterized in that** the solvent is one in which the formed ammonium halide is not readily soluble, and most of the ammonium halide is separated off by filtration or centrifugation prior to ion exchange treatment.

3. Process according to Claim 1 or 2, **characterized in that** the optionally filtered or centrifuged reaction mixture is treated firstly with a strongly acidic cation exchanger and then with a weakly basic anion exchanger.

4. Process according to one of Claims 1 to 3, **characterized in that** the solvent is one from the group consisting of dichloromethane, di-C₁₋₄-alkyl ketones, C₁₋₄-alkyl C₂₋₄-alkanoates, tetrahydrofuran and acetonitrile.

5. Process according to Claim 4, **characterized in that** the solvent is dichloromethane, acetone or butyl acetate.

6. Process according to one of Claims 1 to 5, **characterized in that** an aryl cyanate of the general formula 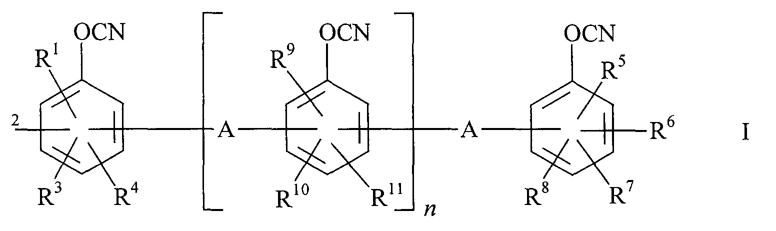 wherein n is an integer from 0 to 20, the symbols A are in each case a direct chemical bond or a divalent group, and R¹ to R¹¹ independently of one another are hydrogen, halogen, C₁₋₄-alkoxy, C₁₋₄-alkylthio, C₁₋₄-alkoxycarbonyl or C₁₋₄-alkyl which is optionally completely or partially substituted by fluorine, is prepared.

## Revendications

1. Procédé de préparation d'arylcyanates par réaction de phénols polyhydriques avec un halogénure de cyanogène en présence d'une amine tertiaire et d'un solvant non aqueux à une faible température et ensuite élimination de l'halogénure d'ammonium tertiaire formé du mélange réactionnel, **caractérisé en ce que** l'élimination de l'halogénure d'ammonium est réalisée entièrement ou partiellement par traitement du mélange réactionnel non aqueux avec des échangeurs de cations et d'anions.

2. Procédé selon la revendication 1, **caractérisé en ce que** le solvant utilisé est un solvant dans lequel l'halogénure d'ammonium formé est difficilement soluble et la majeure partie de l'halogénure d'ammonium est séparée par filtration ou centrifugation avant le traitement par échangeurs d'ions.

3. Procédé selon la revendication 1 ou 2, **caractérisé en ce que** le mélange réactionnel éventuellement filtré ou centrifugé est d'abord traité par un échangeur de cations fortement acide et ensuite par un échangeur d'anions, faiblement basique.

4. Procédé selon l'une quelconque des revendications 1 à 3, **caractérisé en ce que** le solvant utilisé est un solvant choisi parmi le groupe constitué de dichlorométane, de di-C₁₋₄-alkylcétones, de C₁₋₄-alkyl-C₂₋₄-alcanoates, du tétrahydrofurane et de l'acétonitrile.

5. Procédé selon la revendication 4, **caractérisé en ce que** le solvant utilisé est le dichlorométhane, l'acétone ou l'acétate de butyle.

6. Procédé selon l'une quelconque des revendications 1 à 5, **caractérisé en ce que** l'on prépare un arylcyanate de formule générale 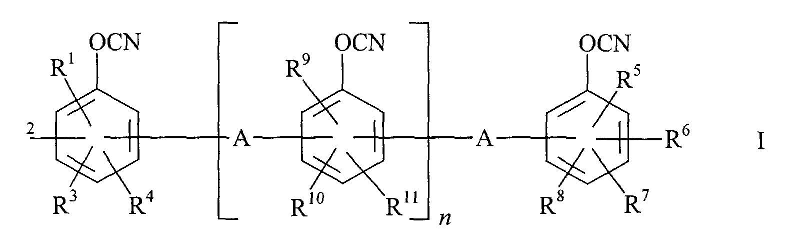 dans laquelle n est un entier de 0 à 20, le symbole A désigne dans chaque cas une liaison chimique directe ou un groupe bivalent, et R¹ à R¹¹ sont, indépendemment les uns des autres, un hydrogène, un halogène, un alcoxy en C₁₋₄, un alkylthio en C₁₋₄, un alcoxycarbonyle en C₁₋₄ ou un alkyle en C₁₋₄ éventuellement entièrement ou partiellement fluorosubstitué.
